(19) Europäisches Patentamt
European Patent Office
Office européen des brevets

(11) **EP 3 342 361 A1**

(12) **EUROPEAN PATENT APPLICATION**
published in accordance with Art. 153(4) EPC

(43) Date of publication:
**04.07.2018 Bulletin 2018/27**

(51) Int Cl.:
***A61B 17/68*** *(2006.01)*

(21) Application number: **16823674.3**

(86) International application number:
**PCT/CN2016/077115**

(22) Date of filing: **23.03.2016**

(87) International publication number:
**WO 2017/008527 (19.01.2017 Gazette 2017/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **15.07.2015 CN 201510414019**

(71) Applicant: **Fujian Normal University**
**Fuzhou, Fujian 350100 (CN)**

(72) Inventors:
• **FAN, Yifang**
  **Fuzhou**
  **Fujian 350100 (CN)**
• **FAN, Yubo**
  **Fuzhou**
  **Fujian 350100 (CN)**
• **LI, Zhiyu**
  **Fuzhou**
  **Fujian 350100 (CN)**

(74) Representative: **Gulde & Partner**
**Patent- und Rechtsanwaltskanzlei mbB**
**Wallstraße 58/59**
**10179 Berlin (DE)**

(54) **FORMING METHOD FOR ORTHOPEDIC INTERNAL FIXATION**

(57) The present invention relates to the field of orthopedics instrument, and aims to provide a method for forming an orthopedic internal fixation object. In the method for forming the orthopedic internal fixation material, the geometric relationship between the fixation object and the host bone is determined through reverse engineering so that a high degree of fitting is achieved between the fixation object and the host bone, and the stress shielding is reduced, the fixation object is designed into a mortise-tenon structure including a mortise portion, a tenon portion and a dovetail slot plug, to avoid damage to the host bone and reduce stress concentration. The beneficial effects of the present invention lie in that the present invention creatively proposes an individualized forming method for an orthopedic passive internal fixation object. Compared with the prior art, the orthopedic internal fixation object according to the present invention are fixed to the host bone by a mortise and tenon structure to be free from relying on the screw, thus avoiding damage to the host bone, and avoiding the concentration of stress in the fixation process.

**EP 3 342 361 A1**

Fig 1

**Description**

**FIELD OF THE INVENTION**

The present invention relates to the field of orthopedic instruments, and in particular to a method for forming an orthopedic internal fixation object.

**BACKGROUND OF THE INVENTION**

Orthopedics internal fixation technology has been widely used in clinical practice. Outstanding orthopedists are often not only a superb "Luban" (an ancient Chinese master of carpenter. It is now usually used to describe a specialist), but also an excellent inventor. Based on the structures such as rod-screw and plate-screw, mechanical problems such as the fixation, stress and durability of an orthopedic implant are solved. Regarding the orthopedic implant fixed by screws, since the fixation of the screw with the host bone is destructive, the industrialized structures such as rod, plate, band and wire cannot fit the surface of the individualized host bone, and modulus of elasticity of the implant is different from the bone *in vivo* and for other reasons, after the implant is fixed, mechanical phenomena such as "stress concentration" and "stress shielding" may occur to the host bone, resulting in occurrence of "bone atrophy", "bone nonunion" and other complications.

Therefore, it is a key technology that needs breaking through urgently to create individualized passive internal fixation object based on reverse and forward design through 3D printing, which is free from the reliance on the screws when to fix the orthopedic implants, so as to avoid stress concentration on the host bone and reduce stress shielding. This not only can make new progress in the theoretical research of biomechanics of bone and in the method of forward and reverse engineering design of orthopedic passive implants, but also is expected to be applied to clinic. With the aging of the population, the continuous growth of GDP and improvement of medical policies, China's orthopedic instrument market continues to grow at a rate of 15% to 20% annually.

**SUMMARY OF THE INVENTION**

An objective of the present invention is to overcome the above defects and provide a method for forming an orthopedic internal fixation object that can overcome stress concentration and stress shielding.

In order to solve the above technical problems, the technical solution adopted by the present invention is as follows:

A method for forming an orthopedic internal fixation object, includes the following steps S1 to S5.

In step 1, continuous tomographic images of a bone *in vivo* are obtained by tomography, and the continuous tomographic images are stacked in a reverse engineering software, and a three-dimensional model of the bone *in vivo* is reconstructed in a scan coordinate system.

In step 2, the centroid and a principal axis of inertia of the bone *in vivo* are calculated, to obtain the density $\rho$ of the volume element, and a horizontal resolution $\Delta$x, a vertical resolution $\Delta$y and a layer distance $\Delta$z of the tomographic images are obtained, $\Delta V = \Delta x \Delta y \Delta z$, and the position coordinates $(x_{oi}, y_{oi}, z_{oi})$ of a point i of the bone *in vivo* in the scan coordinate system are obtained, and the above parameters are substituted into the formula.

$$\begin{cases} \alpha = \dfrac{1}{2}\arctan\left(\dfrac{2\sum y_{oi}z_{oi}\rho_i\Delta V}{\sum y_{oi}^2\rho_i\Delta V - \sum z_{oi}^2\rho_i\Delta V}\right) \\[3ex] \beta = \dfrac{1}{2}\arctan\left(\dfrac{2\sum x_{\alpha i}z_{\alpha i}\rho_i\Delta V}{\sum x_{\alpha i}^2\rho_i\Delta V - \sum z_{\alpha i}^2\rho_i\Delta V}\right) \\[3ex] \gamma = \dfrac{1}{2}\arctan\left(\dfrac{2\sum x_{\beta i}y_{\beta i}\rho_i\Delta V}{\sum x_{\beta i}^2\rho_i\Delta V - \sum y_{\beta i}^2\rho_i\Delta V}\right) \end{cases} \quad ,$$

calculate to obtain $\alpha$, $\beta$ and $\gamma$, where $(x_{\alpha i}, y_{\alpha i}, z_{\alpha i})$ are position coordinates of the point i on the bone *in vivo* after rotating about the x-axis by an angle $\alpha$, and $(x_{\beta i}, y_{\beta i}, z_{\beta i})$ are position coordinates of the point i on the bone *in vivo* after rotating about x-axis by the angle $\alpha$ and then rotating about y-axis by an angle $\beta$.

In step 3, the scan coordinate system is rotated about the x-axis by the angle $\alpha$, and is rotated about the y-axis by the angle $\beta$, and is rotated then about a Z-axis by an angle $\gamma$, to move the origin of the scan coordinate system to the centroid of the bone *in vivo* to realize posture positioning of the bone *in vivo*.

In step 4, coordinates $(x_i, y_i, z_i)$ of the point i on the bone *in vivo* surface in step 3 are obtained, and are substituted into the arithmetic formula:

$$\begin{cases} x_i^{'} = D_x(x_i, s, c) \\ y_i^{'} = D_y(y_i, s, c), \\ z_i^{'} = D_z(z_i, s, c) \end{cases}$$

to obtain coordinates $(X_i^{'}, Y_i^{'}, Z_i^{'})$ of the point i on the enlarged bone *in vivo*, where $D_X(x_i, s, c)$, $D_Y(y_i, s, c)$, $D_Z(z_i, s, c)$ represent quadratrix, s represents the enlargement factor, c represents the convexity and concavity of the position curve where the point i is located, and then calculates to obtain the three dimensional model of the enlarged bone *in vivo*, to do "or" Boolean operation between the enlarged bone *in vivo* and the bone *in vivo*, to obtain a shell of the bone *in vivo*.

In step 5, the shell of the bone *in vivo* in step 4 is cut to be designed into a mortise-tenon structure including a mortise portion, a tenon portion and a dovetail slot plug.

The beneficial effects of the present invention lie in that the present invention creatively proposes an individualized forming method for an orthopedic passive internal fixation object. Compared with the prior art, the orthopedic internal fixation object according to the present invention adopts a structure with mortise and tenon to achieve the fixation and be free from relying on the screw, which avoids damage to the host bone, and avoids the occurrence of stress concentration in the fixation process; the geometric relationship between the fixation object and the host bone is from the reverse engineering, such that the fixation object and the host bone are fitted to each other in a high degree, thereby reducing the stress shielding between the fixation object and the host bone; these two techniques eliminate the occurrence of bone atrophy, bone nonunion, and other complications from the source.

## BRIEF DESCRIPTION OF THE ACCOMPANYING DRAWINGS

Figure 1 is a flowchart of a method for forming an orthopedic internal fixation object according to an embodiment of the present invention;

Figure 2 is a schematic view showing a three-dimensional reconstruction of a first metatarsus according to an embodiment of the present invention;

Figure 3 is a state diagram of the first metatarsus according to an embodiment of the present invention before its posture is fixed;

Figure 4 is a state diagram of the first metatarsus according to an embodiment of the present invention after its posture is fixed;

Figure 5 is a comparison diagram showing the first metatarsus according to an embodiment of the present invention before and after its posture is fixed;

Figure 6 is a perspective view of a first metatarsus shell according to an embodiment of the present invention;

Figure 7 is a front view of the first metatarsus shell according to an embodiment of the present invention;

Figure 8 is a top view of the first metatarsus shell according to an embodiment of the present invention;

Figure 9 is a view showing the combination of the first metatarsus shell and the first metatarsus according to an embodiment of the present invention; and

Figure 10 is a schematic view showing the structure of the first metatarsus shell with mortise and tenon according to an embodiment of the present invention.

## DETAILED DESCRIPTION OF THE PREFERRED EMBODIMENTS

In order to describe in detail the technical content, the structural feature, the achieved object and effect of the present invention, the present invention is described in detail in conjunction with embodiments and drawings.

The most important idea of the present invention lies in that: a geometric relationship between the fixation object and the host bone is determined through reverse engineering so that a high degree of conformity is achieved between the fixation object and the host bone to reduce the stress shielding; the fixation object is designed into a mortise-tenon structure including a mortise portion, a tenon portion and a dovetail slot plug, to avoid damage to the host bone, and reduce stress concentration.

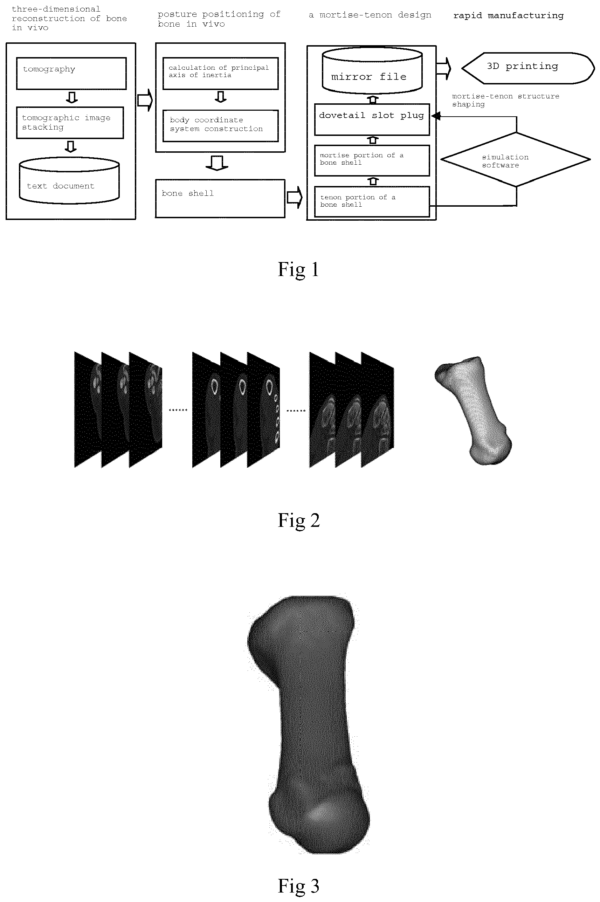

Referring to Figure 1, the method for forming the orthopedic internal fixation object according to this embodiment includes the following steps 1 to 5.

In step 1, continuous tomographic images of a bone *in vivo* are obtained by tomography, and the continuous tomographic images are stacked in a reverse engineering software, and a three-dimensional model of the bone *in vivo* is reconstructed in a scan coordinate system.

In step 2, the centroid and a principal axis of inertia of the bone *in vivo* are calculated, to obtain the density $\rho$ of the volume element, and a horizontal resolution $\Delta x$, a vertical resolution $\Delta y$ and a layer distance $\Delta z$ of the tomographic images are obtained, $\Delta V = \Delta x \Delta y \Delta z$, and the position coordinates $(x_{oi}, y_{oi}, z_{oi})$ of a point i of the bone *in vivo* in the scan coordinate system are obtained, and the above parameters are substituted into the formula.

$$\begin{cases} \alpha = \dfrac{1}{2}\arctan\left(\dfrac{2\sum y_{oi}z_{oi}\rho_i\Delta V}{\sum y_{oi}^2\rho_i\Delta V - \sum z_{oi}^2\rho_i\Delta V}\right) \\[2ex] \beta = \dfrac{1}{2}\arctan\left(\dfrac{2\sum x_{\alpha i}z_{\alpha i}\rho_i\Delta V}{\sum x_{\alpha i}^2\rho_i\Delta V - \sum z_{\alpha i}^2\rho_i\Delta V}\right) \\[2ex] \gamma = \dfrac{1}{2}\arctan\left(\dfrac{2\sum x_{\beta i}y_{\beta i}\rho_i\Delta V}{\sum x_{\beta i}^2\rho_i\Delta V - \sum y_{\beta i}^2\rho_i\Delta V}\right) \end{cases} ,$$

calculate to obtain $\alpha$, $\beta$ and $\gamma$, where $(x_{\alpha i}, y_{\alpha i}, z_{\alpha i})$ are position coordinates of the point i on the bone *in vivo* after rotating about the x-axis by an angle $\alpha$, and $(x_{\beta i}, y_{\beta i}, z_{\beta i})$ are position coordinates of the point i on the bone *in vivo* after rotating about x-axis by the angle $\alpha$ and then rotating about y-axis by an angle $\beta$.

In step 3, the scan coordinate system is rotated about the x-axis by the angle $\alpha$, and is rotated about the y-axis by the angle $\beta$, and is rotated then about a Z-axis by an angle $\gamma$, to move the origin of the scan coordinate system to the centroid of the bone *in vivo* to realize posture positioning of the bone *in vivo*.

In step 4, coordinates $(x_i, y_i, z_i)$ of the point i on the bone *in vivo* surface in step 3 are obtained, and are substituted into the arithmetic formula:

$$\begin{cases} x_i' = D_x(x_i, s, c) \\ y_i' = D_y(y_i, s, c), \\ z_i' = D_z(z_i, s, c) \end{cases}$$

to obtain coordinates $(X_i', Y_i', Z_i')$ of the point i on the enlarged bone *in vivo,* where $D_X(x_i, s, c)$, $D_Y(y_i, s, c)$, $D_Z(z_i, s, c)$ represent quadratrix, s represents the enlargement factor, c represents the convexity and concavity of the position curve where the point i is located, and then calculates to obtain the three dimensional model of the enlarged bone *in vivo,* to do "or" Boolean operation between the enlarged bone *in vivo* and the bone *in vivo,* to obtain a shell of the bone *in vivo*.

In step 5, the shell of the bone *in vivo* in step 4 is cut to be designed into a mortise-tenon structure including a mortise portion, a tenon portion and a dovetail slot plug.

It can be seen from the above description that the beneficial effects of the present invention lie in that the present invention creatively proposes an individualized forming method for an orthopedic passive internal fixation object. Compared with the prior art, the orthopedic internal fixation object according to the present invention adopts a mortise-tenon structure to achieve the fixation and be free from relying on the screw, which avoids damage to the host bone, and avoids the occurrence of stress concentration in the fixation process; the geometric relationship between the fixation object and the host bone is from the reverse engineering, such that the fixation object and the host bone are fitted to each other in a high degree, thereby reducing the stress shielding between the fixation object and the host bone; these two techniques eliminate the occurrence of bone atrophy, bone nonunion, and other complications from the source.

Further, between step 4 and step 5, the method further includes: determining the thicknesses of the shell of the bone *in vivo* at various points through the elastic modulus calculation and the mechanics calculation of the bone *in vivo* and the internal fixation object of the bone.

As can be seen from the above description that, since the thickness of the bone shell is non-uniform, the thickness of the bone shell at each point is determined according to the thickness of the compact bone from the point to the principal axis, and the strength and durability analysis are performed according to the elastic modulus of the compact bone and the internal fixation material (titanium alloy) in conjunction with the mechanics simulation analysis to determine the final mechanics structure of the internal fixation object.

Further, after step 5, the method further includes: manufacturing the mortise portion, the tenon portion, the dovetail slot plug by 3D printing technology.

It can be seen from the above description that the 3D printing technology has advantages of a low cost, a fast forming speed, a high forming precision and unlimited shapes, and is suitable for the production of the fixation object in the invention and can be adapted to the needs of different patients.

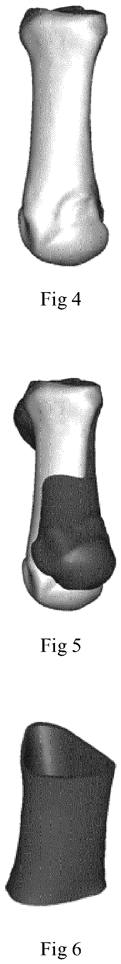

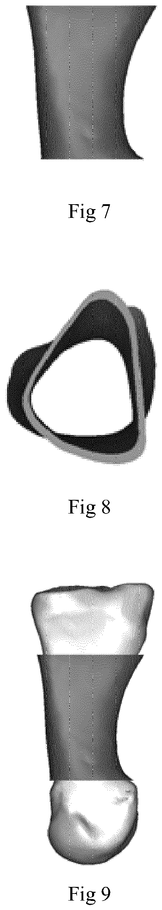

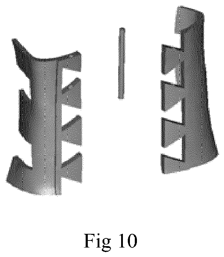

Reference is made to Figure 2 to Figure 10, the first embodiment of the present invention is as follows.

Scanning and reconstructing, positioning, enlarging, cutting, mortise-tenon designing and mirror processing to the healthy side first metatarsus include the following specific steps:

(1) Three-dimensional reconstruction of the first metatarsus on the left: continuous anatomical images of the healthy side first metatarsus are acquired by tomography, and the continuous anatomical images are stacked in the image reverse engineering software to achieve the three-dimensional reconstruction of the first metatarsus.

(2) Posture positioning of the first metatarsus on the left: the centroid and the principal axis of inertia of the first metatarsus are calculated. In the Cartesian coordinate system, the scanning axis of the first metatarsus and two other coordinate axes perpendicular to it are respectively turned to the principal axis of inertia, the origin of the coordinate system moves to the centroid of the first metatarsus to complete the posture positioning. The angular displacement between the first metatarsus scan coordinate system and the principal axis of inertia is calculated from the following equation:

$$\begin{cases} \alpha = \dfrac{1}{2}\arctan\left(\dfrac{2\sum y_{oi}z_{oi}\rho_i\Delta V}{\sum y_{oi}^2\rho_i\Delta V - \sum z_{oi}^2\rho_i\Delta V}\right) \\[3mm] \beta = \dfrac{1}{2}\arctan\left(\dfrac{2\sum x_{\alpha i}z_{\alpha i}\rho_i\Delta V}{\sum x_{\alpha i}^2\rho_i\Delta V - \sum z_{\alpha i}^2\rho_i\Delta V}\right) \\[3mm] \gamma = \dfrac{1}{2}\arctan\left(\dfrac{2\sum x_{\beta i}y_{\beta i}\rho_i\Delta V}{\sum x_{\beta i}^2\rho_i\Delta V - \sum y_{\beta i}^2\rho_i\Delta V}\right) \end{cases}$$

where $\rho$ denotes the density of the volume element, $\Delta V=\Delta x\Delta y\Delta z$, $\Delta x$, $\Delta y$ and $\Delta z$ are respectively the horizontal resolution, vertical resolution and layer distance of the tomographic images, respectively, and $\alpha$, $\beta$ and $\gamma$ respectively represent the angular displacement of the rotation about the x-axis, y-axis and z-axis, $(x_{oi}, y_{oi}, z_{oi})$ are the position coordinates of the point i on the first metatarsus in the scan coordinate system, $(x_{\alpha i}, y_{\alpha i}, z_{\alpha i})$ are the position coordinates of the point i on the first metatarsus after the first metatarsus is rotated by the angle $\alpha$ about the x-axis, $(x_{\beta i}, y_{\beta i}, z_{\beta i})$ are the position coordinates of the point i on the first metatarsus after the first metatarsus is rotated by an angle $\alpha$ about the x-axis and then rotated by an angle β about the y-axis.

(3) Enlarge and Boolean operations: for the first metatarsus after being fixed in position, the first metatarsus shell is obtained by enlarging the first metatarsus with a quadratrix and doing an "OR" Boolean operation between the quadratrix with the first metatarsus. The convex-concave function is enlarged by the following method:

$$\begin{cases} x_i' = D_x(x_i, s, c) \\ y_i' = D_y(y_i, s, c) \\ z_i' = D_z(z_i, s, c) \end{cases}.$$

In the formula, $(x_i, y_i, z_i)$ respectively represent the position coordinates of the point i on the surface of the first metatarsus after the first metatarsus is rotated by the angles $\alpha$, $\beta$, and $\gamma$ about the x-axis, y-axis, and z-axis, $D_X(x_i, s, c)$, $D_y(y_i, s, c)$, $D_z(z_i, s, c)$ represent quadratrix, s represents the enlargement factor, and c represents the concavity and convexity of the position curve where the point i is located.

(4) Mortise and tenon design: according to the situations such as the injury state and the fixing position of the first phalanx on the right side, the bone shell is cut and designed into a mortise-tenon structure including a mortise portion, a tenon portion and a dovetail slot plug structure.

(5) Simulation calculation: the thickness of the bone shell is non-uniform, and the thickness of the bone shell at each point is determined according to the thickness of the compact bone between the point and the principal axis. Analysis of strength and durability is performed according to the elastic modulus of the compact bone and the material of the internal fixation object (titanium alloy) in combination with the mechanics simulation analysis, to determine the final mechanics structure of the internal fixation object.

By this embodiment, tomographic images of the healthy side bone *in vivo* are obtained by scanning, and the reconstructed healthy side bone *in vivo* is subjected to steps such as positioning, enlarging, Boolean operating and mortar-and-tenon structural designing, mirror processing and rapid manufacturing to obtain an internal fixation object fitted with and coupled with, in mechanics aspect, the surface of the host bone. A bone shell fitted with the surface of the unhealthy side bone is obtained based on the healthy side bone, and through the mortar-tenon design, it gets rid of the reliance of the orthopedic internal fixation object on the screw, thereby allowing the bone internal fixation object not to cause "stress concentration" to the host bone, to reduce the "stress shielding". This is just the innovation of the present invention.

To sum up, in the method for forming an orthopedic internal fixation object provided by the present invention, the orthopedic internal fixation object adopts the mortise-tenon structure to be fixed to the host bone, thus avoiding the reliance on the screw, avoiding the damage to the host bone, and also avoiding the occurrence of stress concentration in the fixation process. The geometric relationship between the fixation object and the host bone comes from a reverse engineering that results in a high degree of fit between the fixation object and the host bone, thereby reducing the stress shielding between the fixation object and the host bone. The two techniques eliminate the occurrence of bone atrophy, bone nonunion and other complications from the source. Since the thickness of the bone shell is non-uniform, and the thickness at each point is determined according to the thickness of the compact bone between the point and the principal axis, and the strength and durability analysis are performed according to the elastic modulus of the compact bone and the material of the internal fixation object in combination with the mechanics simulation, to determine the final mechanics structure of the internal fixation object, and moreover, using the 3D printing technology, the cost is low, the forming speed is fast, the forming precision is high, and the shape is not limited, therefore, 3D printing is bound to play an important role in individualized medical treatment.

The above is only the embodiments of the present invention, and does not intend to limit the scope of the present invention. All equivalent structures or equivalent process changes made by the specification and the accompanying drawings of the present invention or directly or indirectly used in other related technologies are all included in the protection scope of the present invention.

## Claims

1. A method for forming an orthopedic internal fixation object, **characterized by** comprising:

step 1, obtaining continuous tomographic images of a bone *in vivo* by tomography, stacking the continuous tomographic images in a reverse engineering software, and reconstructing a three-dimensional model of the bone *in vivo* in a scan coordinate system;

step 2, calculating the centroid and a principal axis of inertia of the bone *in vivo,* to obtain the density $\rho$ of the volume element, obtaining a horizontal resolution $\Delta x$, a vertical resolution $\Delta y$ and a layer distance $\Delta z$ of the tomographic images, $\Delta V = \Delta x \Delta y \Delta z$, obtaining the position coordinates $(x_{oi}, y_{oi}, z_{oi})$ of a point i of the bone *in vivo* in the scan coordinate system, and substituting the above parameters into the formula:

$$\begin{cases} \alpha = \dfrac{1}{2}\arctan\left(\dfrac{2\sum y_{oi}z_{oi}\rho_i\Delta V}{\sum y_{oi}^2\rho_i\Delta V - \sum z_{oi}^2\rho_i\Delta V}\right) \\[2ex] \beta = \dfrac{1}{2}\arctan\left(\dfrac{2\sum x_{\alpha i}z_{\alpha i}\rho_i\Delta V}{\sum x_{\alpha i}^2\rho_i\Delta V - \sum z_{\alpha i}^2\rho_i\Delta V}\right) \\[2ex] \gamma = \dfrac{1}{2}\arctan\left(\dfrac{2\sum x_{\beta i}y_{\beta i}\rho_i\Delta V}{\sum x_{\beta i}^2\rho_i\Delta V - \sum y_{\beta i}^2\rho_i\Delta V}\right) \end{cases},$$

calculating to obtain $\alpha$, $\beta$ and $\gamma$, wherein $(x_{\alpha i}, y_{\alpha i}, z_{\alpha i})$ are the position coordinates of the point i on the bone *in vivo* after the bone *in vivo* rotates about the x-axis by an angle $\alpha$, and $(x_{\beta i}, y_{\beta i}, z_{\beta i})$ are position coordinates of the point i on the bone *in vivo* after the bone *in vivo* rotates about the x-axis by the angle $\alpha$ and then rotates about a y-axis by an angle $\beta$;

step 3, rotating the scan coordinate system about the x-axis by the angle $\alpha$, and rotating the scan coordinate system about the y-axis by the angle $\beta$, and rotating the scan coordinate system about the z-axis by an angle $\gamma$, to move the origin of the scan coordinate system to the centroid of the bone *in vivo* to realize posture positioning of the bone *in vivo*;

step 4, obtaining the coordinates $(x_i, y_i, z_i)$ of the point i on the surface of the bone *in vivo* in step 3, and substituting the coordinates into the formula:

$$
\begin{cases}
x_i' = D_x(x_i, s, c) \\
y_i' = D_y(y_i, s, c) \\
z_i' = D_z(z_i, s, c)
\end{cases}
$$

to obtain the coordinates $(X_i', Y_i', Z_i')$ of the point i on an enlarged bone *in vivo*, wherein $D_X(x_i, s, c)$, $D_Y(y_i, s, c)$, $D_Z(z_i, s, c)$ represent quadratrix, s represents an enlargement factor, c represents the convexity and concavity of the position curve where the point i is located, and then calculates to obtain the three dimensional model of the enlarged bone *in vivo*, to do "or" Boolean operations between the enlarged bone *in vivo* and the bone *in vivo,* to obtain a shell of the bone *in vivo*;

step 5, cutting and designing the shell of the bone *in vivo* in step 4 into a mortise-tenon structure including a mortise portion, a tenon portion and a dovetail slot plug.

2.  The method for forming the orthopedic internal fixation object according to claim 1, further comprising, between step 4 and step 5,
    determining the thickness of the shell of the bone *in vivo* at various points through the elastic modulus calculation and the mechanics calculation of the bone *in vivo* and the internal fixation object of the bone *in vivo*.

3.  The method for forming the orthopedic internal fixation object according to claim 1, further comprising: after step 5, manufacturing a tenon portion, a dorsal portion and a dovetail slot plug by 3D printing technology.

three-dimensional
reconstruction of bone
in vivo

posture positioning of
bone in vivo

a mortise-tenon design

**rapid manufacturing**

| tomography |
| --- |

⇩

| tomographic image stacking |
| --- |

⇩

text document

⇨

| calculation of principal axis of inertia |
| --- |

⇩

| body coordinate system construction |
| --- |

⇩

| bone shell |
| --- |

⇨

mirror file

⇧

| dovetail slot plug |
| --- |

⇧

| mortise portion of a bone shell |
| --- |

⇧

| tenon portion of a bone shell |
| --- |

⇨ 3D printing

mortise-tenon structure
shaping

simulation
software

Fig 1

Fig 2

Fig 3

Fig 4

Fig 5

Fig 6

Fig 7

Fig 8

Fig 9

Fig 10

# INTERNATIONAL SEARCH REPORT

International application No.

**PCT/CN2016/077115**

## A. CLASSIFICATION OF SUBJECT MATTER

A61B 17/68 (2006.01) i

According to International Patent Classification (IPC) or to both national classification and IPC

## B. FIELDS SEARCHED

Minimum documentation searched (classification system followed by classification symbols)

A61B; A61F 2

Documentation searched other than minimum documentation to the extent that such documents are included in the fields searched

Electronic data base consulted during the international search (name of data base and, where practicable, search terms used)

CNABS, YEN, CNTXT: CT print mortise tenon bone osteo+ computed algorithm

## C. DOCUMENTS CONSIDERED TO BE RELEVANT

| Category* | Citation of document, with indication, where appropriate, of the relevant passages | Relevant to claim No. |
|---|---|---|
| PX | CN 105055006 A (FUJIAN NORMAL UNIVERSITY), 18 November 2015 (18.11.2015), claims 1-3 | 1-3 |
| A | CN 204379403 U (GUANGZHOU BELLOWS BIOTECHNOLOGY CO., LTD.), 10 June 2015 (10.06.2015), description, paragraphs [0027]-[0038], and figures 1-9 | 1-3 |
| A | CN 103860293 A (PEKING UNIVERSITY THIRD HOSPITAL; SOUTH CHINA UNIVERSITY OF TECHNOLOGY), 18 June 2014 (18.06.2014), the whole document | 1-3 |
| A | CN 104136199 A (STRATASYS LTD.), 05 November 2014 (05.11.2014), the whole document | 1-3 |
| A | US 2006276925 A1 (UNIV MICHIGAN), 07 December 2006 (07.12.2006), the whole document | 1-3 |

☐ Further documents are listed in the continuation of Box C. ☒ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 16 June 2016 (16.06.2016) | **30 June 2016 (30.06.2016)** |

| Name and mailing address of the ISA/CN:<br>State Intellectual Property Office of the P. R. China<br>No. 6, Xitucheng Road, Jimenqiao<br>Haidian District, Beijing 100088, China<br>Facsimile No.: (86-10) 62019451 | Authorized officer<br><br>**FAN, Wenyang**<br><br>Telephone No.: (86-10) **62085628** |

Form PCT/ISA/210 (second sheet) (July 2009)

## INTERNATIONAL SEARCH REPORT
### Information on patent family members

International application No.

**PCT/CN2016/077115**

| Patent Documents referred in the Report | Publication Date | Patent Family | Publication Date |
|---|---|---|---|
| CN 105055006 A | 18 November 2015 | None | |
| CN 204379403 U | 10 June 2015 | None | |
| CN 103860293 A | 18 June 2014 | CN 103860293 B | 06 April 2016 |
| CN 104136199 A | 05 November 2014 | US 2014312535 A1 | 23 October 2014 |
| | | JP 2014533613 A | 15 December 2014 |
| | | EP 2780154 A1 | 24 September 2014 |
| | | HK 1203172 A1 | 23 October 2015 |
| | | KR 20140097378 A | 06 August 2014 |
| | | WO 2013072874 A1 | 23 May 2013 |
| | | IL 234456 A1 | 30 October 2014 |
| US 2006276925 A1 | 07 December 2006 | US 7509183 B2 | 24 March 2009 |
| | | WO 2004093657 A2 | 04 November 2004 |

Form PCT/ISA/210 (patent family annex) (July 2009)